(19) Europäisches Patentamt
European Patent Office
Office européen des brevets

(11) EP 3 323 418 A1

(12) # EUROPEAN PATENT APPLICATION

(43) Date of publication:
**23.05.2018 Bulletin 2018/21**

(21) Application number: **17206293.7**

(22) Date of filing: **14.05.2013**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority:
**14.05.2012 US 201261646538 P**
**15.05.2012 US 201261647478 P**
**01.06.2012 US 201261654383 P**
**01.06.2012 US 201261654292 P**
**05.06.2012 US 201261655541 P**
**05.06.2012 US 201261655527 P**
**07.02.2013 US 201361762225 P**
**14.02.2013 US 201361764563 P**
**21.02.2013 US 201361767676 P**
**21.02.2013 US 201361767647 P**
**20.03.2013 US 201361803721 P**

(62) Document number(s) of the earlier application(s) in accordance with Art. 76 EPC:
**13791356.2 / 2 849 758**

(51) Int Cl.:
***A61K 31/675*** *(2006.01)* ***A61P 29/00*** *(2006.01)*

(71) Applicant: **Antecip Bioventures II LLC**
**New York, NY 10111 (US)**

(72) Inventor: **Tabuteau, Herriot**
**New York, NY New York 10010 (US)**

(74) Representative: **Penty Geraets, Edith Anna**
**Urquhart-Dykes & Lord LLP**
**Euston House**
**24 Eversholt Street**
**London NW1 1AD (GB)**

Remarks:
This application was filed on 08.12.2017 as a divisional application to the application mentioned under INID code 62.

(54) **COMPOSITIONS COMPRISING ZOLEDRONIC ACID OR RELATED COMPOUNDS FOR RELIEVING INFLAMMATORY PAIN AND RELATED CONDITIONS**

(57) Oral dosage forms of bisphosphonate compounds, such as zoledronic acid, can be used to treat or alleviate pain or related conditions. Although an oral dosage form with enhanced bioavailability with respect to the bisphosphonate compound can be used, the treatment can also be effective using an oral dosage form that includes a bisphosphonate compound, such as zoledronic acid, wherein the bioavailability of the bisphosphonate is unenhanced, or is substantially unenhanced.

EP 3 323 418 A1

## Description

CROSS-REFERENCE TO RELATED APPLICATIONS

This application claims the benefit of United States Provisional Applications 61/646,538, filed May 14, 2012; 61/647,478, filed May 15, 2012; 61/654,292, filed June 1, 2012; 61/654,383, filed June 1, 2012; 61/655,527, filed June 5, 2012; 61/655,541, filed June 5, 2012; 61/764,563, filed February 4, 2013; 61/762,225, filed February 7, 2013; 61/767,647, filed February 21, 2013; 61/767,676, filed February 21, 2013; and 61/803,721, filed March 20, 2013, all of which are incorporated by reference in their entirety herein.

BACKGROUND

Bisphosphonate compounds are potent inhibitors of osteoclast activity, and are used clinically to treat bone-related conditions such as osteoporosis and Paget's disease of bone; and cancer-related conditions including multiple myeloma, and bone metastases from solid tumors. They generally have low oral bioavailability.

SUMMARY

It has been discovered that oral dosage forms of bisphosphonate compounds, such as zoledronic acid, can be used to treat or alleviate pain or related conditions. Although an oral dosage form with enhanced bioavailability with respect to the bisphosphonate compound can be used, the treatment can also be effective using an oral dosage form that includes a bisphosphonate compound, such as zoledronic acid, wherein the bioavailability of the bisphosphonate is unenhanced, or is substantially unenhanced.

Some embodiments include a method of relieving inflammatory pain comprising administering an oral dosage form containing zoledronic acid to a mammal in need thereof, wherein the mammal experiences significant pain relief more than 3 hours after administration of the dosage form.

Some embodiments include a method of relieving pain associated with an arthritis comprising administering an oral dosage form containing zoledronic acid to a human being in need thereof.

Some embodiments include a method of treating complex regional pain syndrome comprising administering an oral dosage form containing zoledronic acid to a mammal in need thereof.

Some embodiments include an oral dosage form comprising zoledronic acid, wherein the oral bioavailability of zoledronic acid is substantially unenhanced. For example, in some embodiments, the oral bioavailability in the dosage form is about 0.01% to about 4%.

Some embodiments include a pharmaceutical product comprising more than one unit of an oral dosage form described herein. In some embodiments, each unit of the oral dosage form contains about 1 mg to about 50 mg of zoledronic acid.

Some embodiments include a method of relieving inflammatory pain comprising administering an oral dosage form containing zoledronic acid to a mammal in need thereof.

In some embodiments, the mammal receives a total monthly dose of zoledronic acid that is about 800 $mg/m^2$ or less.

In some embodiments, the dosage form contains about 10 $mg/m^2$ to about 20 $mg/m^2$ based upon the body surface area of the mammal.

Some embodiments include a method of relieving inflammatory pain comprising orally administering zoledronic acid to a mammal in need thereof.

In some embodiments, about 300 $mg/m^2$ to about 600 $mg/m^2$ of zoledronic acid is administered per month, based upon the body surface area of the mammal.

In some embodiments, about 50 $mg/m^2$ to about 600 $mg/m^2$ of zoledronic acid is administered per month, based upon the body surface area of the mammal.

BRIEF DESCRIPTION OF DRAWINGS

FIG. 1 is a plot of pain compression thresholds in a rat model of inflammatory pain using three different doses of zoledronic acid. Measurements were taken at baseline (BL) and at various time points after dosing on the days indicated.

FIG. 2A is a graph depicting reversal of arthritis pain for two different doses of zoledronic acid in a rat model of

arthritis pain.

FIG. 2B is a graph depicting pain thresholds for two different doses of zoledronic acid in a rat model of arthritis pain.

FIG. 3 is a graph summarizing the results for vehicle and zoledronic acid treated rats in a rat model of complex regional pain syndrome.

FIG. 4 depicts hindpaw pain thresholds for vehicle and zoledronic acid treated rats in a rat model of complex regional pain syndrome.

FIG. 5 depicts weight bearing for vehicle and zoledronic acid treated rats in a rat model of complex regional pain syndrome.

FIG. 6 depicts paw thickness change for vehicle and zoledronic acid treated rats in a rat model of complex regional pain syndrome.

FIG. 7 depicts the aqueous solubility of disodium zoledronate tetrahydrate as compared to the diacid form of zoledronic acid.

DETAILED DESCRIPTION

Bisphosphonate compounds such as pamidronate or pamidronic acid, neridronate or neridronic acid, olpadronate or olpadronic acid, alendronate or alendronic acid, incadronate or incadronic acid, ibandronate or ibandronic acid, risedronate or risedronic acid, zoledronate or zoledronic acid, etidronate or etidronic acid, clodronate or clodronic acid, tiludronate or tiludronic acid, etc., may be used for a number of medical purposes, such as treatment of undesirable conditions or diseases, including pain relief. This may be accomplished in many instances by administration of oral dosage forms. Generally, an oral dosage form comprising a bisphosphonate such as zoledronic acid is administered orally to a mammal, such as a human being, at least once, to treat a disease or condition, or to relieve pain.

The term "treating" or "treatment" broadly includes any kind of treatment activity, including the diagnosis, cure, mitigation, or prevention of disease in man or other animals, or any activity that otherwise affects the structure or any function of the body of man or other animals.

An oral dosage form of a bisphosphonate such as zoledronic acid may be used to treat, or provide relief of, any type of pain including, but not limited to, inflammatory pain, arthritis pain, complex regional pain syndrome, lumbosacral pain, musculoskeletal pain, neuropathic pain, chronic pain, cancer-related pain, acute pain, postoperative pain, etc. In some instances, pain relief may be palliative, or pain relief may be provided independent of improvement of the disease or condition or the underlying cause of the disease or condition. For example, although the underlying disease may not improve, or may continue to progress, an individual suffering from the disease may experience pain relief.

In some embodiments, the mammal being treated is not suffering from bone metastasis. In some embodiments, the mammal being treated is not suffering from cancer. In some embodiments, the mammal being treated is not suffering from osteoporosis.

For example, zoledronic acid or another bisphosphonate may be administered orally to relieve musculoskeletal pain including low back pain, and pain associated with rheumatoid arthritis, juvenile rheumatoid arthritis, osteoarthritis, erosive osteoarthritis, sero-negative (non-rheumatoid) arthropathies, non-articular rheumatism, peri-articular disorders, axial spondyloarthritis including ankylosing spondylitis, Paget's disease, fibrous dysplasia, SAPHO syndrome, transient osteoarthritis of the hip, vertebral crush fractures, osteoporosis, etc.

In some embodiments, zoledronic acid or another bisphosphonate may also be administered orally to relieve neuropathic pain, including diabetic peripheral neuropathy, post-herpetic neuralgia, trigeminal neuralgia, monoradiculopathies, phantom limb pain, and central pain. Other causes of neuropathic pain include cancer-related pain, lumbar nerve root compression, spinal cord injury, post-stroke pain, central multiple sclerosis pain, HIV-associated neuropathy, and radio-therapy or chemo-therapy associated neuropathy.

In some embodiments, zoledronic acid or another bisphosphonate may be administered orally to relieve inflammatory pain including musculoskeletal pain, arthritis pain, and complex regional pain syndrome.

Examples of musculoskeletal pain include low back pain; and pain associated with vertebral crush fractures, fibrous dysplasia, osteogenesis imperfecta, Paget's disease of bone, transient osteoporosis, and transient osteoporosis of the hip.

Arthritis refers to inflammatory joint diseases that can be associated with pain. Examples of arthritis pain include pain associated with osteoarthritis, erosive osteoarthritis, rheumatoid arthritis, juvenile rheumatoid arthritis, sero-negative (non-rheumatoid) arthropathies, non-articular rheumatism, peri-articular disorders, neuropathic arthropaties including

Charcot's foot, axial spondyloarthritis including ankylosing spondylitis, and SAPHO syndrome.

In some embodiments, zoledronic acid or another bisphosphonate may be administered orally to relieve complex regional pain syndrome, such as complex regional pain syndrome type I (CRPS-I), complex regional pain syndrome type II (CRPS-II), CRPS-NOS, or another type of CRPS. CRPS is a type of inflammatory pain. CRPS can also have a neuropathic component.

Complex regional pain syndrome is a debilitating pain syndrome. It is characterized by severe pain in a limb accompanied by edema, and autonomic, motor and sensory changes.

With respect to use of oral zoledronic acid for relieving pain associated with an inflammatory condition, relief of pain can be short-term, e.g. for a period of hours after administration of the dosage form, and/or relief of pain can be long-term, e.g. lasting for days, weeks, or even months after oral administration of zoledronic acid. In some embodiments, a mammal, such as a human being, experiences significant pain relief at least about 3 hours, at least about 6 hours, at least about 12 hours, at least about 24 hours, at least about 48 hours, at least about one week, at least about 2 weeks, or at least about 3 weeks after administration of an oral dosage form comprising zoledronic acid. In some embodiments, a mammal, such as a human being, experiences significant pain relief during at least part of the time from about 3 hours to about 2 weeks, about 3 hours to about 3 weeks, about 3 hours to about 24 hours, about 6 hours to about 2 weeks, or about 6 hours to about 24 hours, about 3 days to about 2 weeks, about 6 days to about 2 weeks, after administration of an oral dosage form comprising zoledronic acid.

Zoledronic acid or another bisphosphonate may also be administered orally to relieve cancer-related pain, including pain associated with multiple myeloma and bone metastases from solid tumors. In some embodiments, zoledronic acid is used to treat pain that is not cancer-related pain. For example, zoledronic acid may be used to treat pain that is not associated with multiple myeloma, bone metastasis from solid tumors, hypercalcemia of malignancy, giant cell tumor of bone, blood cancers or leukemias, or solid tumors or cancers.

In addition to relieving pain, oral administration of zoledronic acid or another bisphosphonate may also be useful to treat diseases or conditions that may or may not include a pain component. For example, zoledronic acid or another bisphosphonate may be useful to treat any of the pain conditions or types of conditions listed above, including treatment that does not simply relieve the pain of those conditions, and treatment that is carried out in such a way that the condition is treated without pain relief occuring. In addition to any pain relief zoledronic acid or another bisphosphonate may or may not provide, zoledronic acid or another bisphosphonates may be used to treat a disease or condition such as a metabolic disease or condition; an inflammatory disease or condition, including an inflammatory disease or condition that is not associated with pain; a cancer disease or condition; a neurological disease or condition; etc.

In some embodiments, oral administration of zoledronic acid or another bisphosphonate may also be useful to treat complex regional pain syndrome, rheumatoid arthritis, osteoarthritis, erosive osteoarthritis, axial spondyloarthritis including ankylosing spondylitis, acute vertebral crush fracture, fibrous dysplasia, SAPHO syndrome, osteoporosis, transient osteoporosis, or transient osteoporosis of the hip.

In some embodiments, oral administration of zoledronic acid or another bisphosphonate may also be useful to treat hypercalcemia of malignancy, multiple myeloma, bone metastases from solid tumors, Paget's disease of bone, giant cell tumor of bone, blood cancers or leukemias, or solid tumors or cancers.

Zoledronic acid has the structure shown below, and is also referred to as zoledronate.

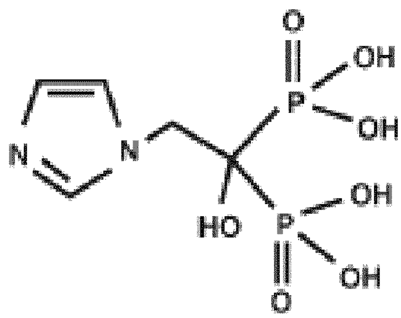

Zoledronic acid

Unless otherwise indicated, any reference to a compound herein, such as zoledronic acid, by structure, name, or any other means, includes pharmaceutically acceptable salts, such as the disodium salt; alternate solid forms, such as polymorphs, solvates, hydrates, etc.; tautomers; or any other chemical species that may rapidly convert to a compound described herein under conditions in which the compounds are used as described herein.

In some embodiments, zoledronic acid is administered in a dosage form comprising a salt form, such as a salt

of a dianion of zoledronic acid. In some embodiments, zoledronic acid is administered in a dosage form comprising a disodium salt form of zoledronic acid. In some embodiments, zoledronic acid is administered in a sodium salt form, such as a monosodium salt, a disodium salt, a trisodium salt, etc. In some circumstances, use of the disodium salt may be desirable. For example, the disodium salt is much more soluble in water than the diacid form. As a result, in some processes, the disodium salt can be easier to work with than the diacid form. Additionally, the sodium salt may be more bioavailable and/or more rapidly absorbed when taken orally as compared to the diacid form.

In some embodiments, zoledronic acid is in a form that has an aqueous solubility, meaning the solubility in water, greater than 1% (w/v), about 5% (w/v) to about 50% (w/v), about 5% (w/v) to about 20% (w/v), about 10% (w/v) to about 15% (w/v), or about 12% (w/v) to about 13% (w/v).

Zoledronic acid or another bisphosphonate may be combined with a pharmaceutical carrier selected on the basis of the chosen route of administration and standard pharmaceutical practice as described, for example, in Remington's Pharmaceutical Sciences, 2005, the disclosure of which is hereby incorporated herein by reference, in its entirety. The relative proportions of active ingredient and carrier may be determined, for example, by the solubility and chemical nature of the compounds, chosen route of administration and standard pharmaceutical practice.

Zoledronic acid or another bisphosphonate may be administered by any means that may result in the contact of the active agent(s) with the desired site or site(s) of action in the body of a patient. The compounds may be administered by any conventional means available for use in conjunction with pharmaceuticals, either as individual therapeutic agents or in a combination of therapeutic agents. For example, they may be administered as the sole active agents in a pharmaceutical composition, or they can be used in combination with other therapeutically active ingredients.

Zoledronic acid or another bisphosphonate may be administered to a human patient in a variety of forms adapted to the chosen route of administration, e.g., orally, rectally, or parenterally. Parenteral administration in this respect includes, but is not limited to, administration by the following routes: pulmonary, intrathecal, intravenous, intramuscular, subcutaneous, intraocular, intrasynovial, transepithelial including transdermal, sublingual and buccal; topically; nasal inhalation via insufflation; and rectal systemic.

The effective amount of zoledronic acid or another bisphosphonate will vary depending on various factors known to the treating physicians, such as the severity of the condition to be treated, route of administration, formulation and dosage forms, physical characteristics of the bisphosphonate compound used, and age, weight and response of the individual patients.

The amount of zoledronic acid or another bisphosphonate in a therapeutic composition may vary. For example, some liquid compositions may comprise about 0.0001% (w/v) to about 50% (w/v), about 0.01% (w/v) to about 20% (w/v), about 0.01% to about 10% (w/v), about 0.001% (w/v) to about 1% (w/v), about 0.1% (w/v) to about 0.5% (w/v), about 1% (w/v) to about 3% (w/v), about 3% (w/v) to about 5% (w/v), about 5% (w/v) to about 7% (w/v), about 7% (w/v) to about 10% (w/v), about 10% (w/v) to about 15% (w/v), about 15% (w/v) to about 20% (w/v), about 20% (w/v) to about 30% (w/v), about 30% (w/v) to about 40% (w/v), or about 40% (w/v) to about 50% (w/v) of zoledronic acid.

Some solid compositions may comprise at least about 5% (w/w), at least about 10% (w/w), at least about 20% (w/w), at least about 50% (w/w), at least about 70% (w/w), at least about 80%, about 10% (w/w) to about 30% (w/w), about 10% (w/w) to about 20% (w/w), about 20% (w/w) to about 30% (w/w), about 30% (w/w) to about 50% (w/w), about 30% (w/w) to about 40% (w/w), about 40% (w/w) to about 50% (w/w), about 50% (w/w) to about 80% (w/w), about 50% (w/w) to about 60% (w/w), about 70% (w/w) to about 75% (w/w), about 70% (w/w) to about 80% (w/w), or about 80% (w/w) to about 90% (w/w) of zoledronic acid.

Any suitable amount of zoledronic acid may be used. Some solid or liquid oral dosage forms, or units of oral dosage forms (referred to collectively herein as "oral dosage form(s)") may contain about 0.005 mg to about 20 mg, about 0.1 mg to about 10 mg, about 0.5 mg to about 10 mg, about 0.2 mg to about 5 mg, about 1 mg to about 500 mg, about 1 mg to about 50 mg, about 10 mg to about 250 mg, about 100 mg to about 300 mg, about 20 mg to about 200 mg, about 20 mg to about 150 mg, about 30 mg to about 100 mg, about 1 mg to about 1,000 mg, about 10 mg to about 50 mg, about 10 mg to about 300 mg, about 10 mg to about 150 mg, about 10 mg to about 100 mg, about 40 mg to about 150 mg, about 10 mg to about 600 mg, about 40 mg to about 600 mg, about 40 mg to about 2000 mg, about 40 mg to about 800 mg, about 25 mg to about 800 mg, about 30 mg to about 800 mg, about 10 mg to about 500 mg, about 50 mg to about 150 mg, about 50 mg, about 100 mg, about 50 mg to about 500 mg, about 100 mg to about 2000 mg, about 300 mg to about 1500 mg, about 200 mg to about 1000 mg, about 100 mg to about 500 mg, or about 150 mg of zoledronic acid, or any amount of zoledronic in a range bounded by, or between, any of these values. In some embodiments, the oral zoledronic acid is administered daily, weekly, monthly, every two or three months, once a year, or twice a year.

In some embodiments, an oral dosage form may contain about 10 $mg/m^2$ to about 20 $mg/m^2$, about 15 $mg/m^2$ to about 20 $mg/m^2$, about 18 $mg/m^2$, about 80 $mg/m^2$ to about 150 $mg/m^2$, about 90 $mg/m^2$ to about 150 $mg/m^2$, about 100 $mg/m^2$ to about 150 $mg/m^2$ of zoledronic acid, or any amount of zoledronic in a range bounded by, or between, any of these values. All dosage ranges or amounts expressed in $mg/m^2$ are based upon the body surface area of the mammal.

In some embodiments the daily oral dose of zoledronic acid is about 0.005 mg to about 20 mg, about 0.1 mg

to about 10 mg, about 0.5 mg to about 10 mg, about 0.2 mg to about 5 mg, or any amount of zoledronic acid in a range bounded by, or between, any of these values. In some embodiments, the daily oral dose of zoledronic acid is less than about 35 $mg/m^2$, less than about 30 $mg/m^2$, less than about 25 $mg/m^2$, about 1 $mg/m^2$ to about 35 $mg/m^2$, about 1 $mg/m^2$ to about 30 $mg/m^2$, about 1.5 $mg/m^2$ to about 25 $mg/m^2$, about 1.8 $mg/m^2$ to about 20 $mg/m^2$, about 10 $mg/m^2$ to about 20 $mg/m^2$, about 10 $mg/m^2$ to about 30 $mg/m^2$, about 15 $mg/m^2$ to about 20 $mg/m^2$, about 18 $mg/m^2$, or any amount of zoledronic acid in a range bounded by, or between, any of these values.

In some embodiments the weekly oral dose of zoledronic acid is about 1 mg to about 1000 mg, about 1 mg to about 500 mg, about 10 mg to about 250 mg, about 100 mg to about 300 mg, about 10 mg to about 100 mg, about 10 mg to about 150 mg, about 10 mg to about 100 mg, about 10 mg to about 300 mg, about 20 mg to about 150 mg, or about 30 mg to about 100 mg. In some embodiments, the weeky oral dose of zoledronic acid is less than about 250 $mg/m^2$, less than about 200 $mg/m^2$, less than about 175 $mg/m^2$, about 6 $mg/m^2$ to about 250 $mg/m^2$, about 10 $mg/m^2$ to about 210 $mg/m^2$, about 10 $mg/m^2$ to about 170 $mg/m^2$, about 4 $mg/m^2$ to about 140 $mg/m^2$, about 100 $mg/m^2$ to about 140 $mg/m^2$, about 126 $mg/m^2$, or any amount of zoledronic acid in a range bounded by, or between, any of these values. The weekly oral dose may be given as a single dose, given once during the week, or may be given in 2, 3, 4, 5, 6, or 7 individual doses during the week.

In some embodiments, the monthly dose of zoledronic acid, or the amount of zoledronic acid that is administered over a period of a month, is about 5000 mg or less, about 4000 mg or less, about 3000 mg or less, about 2000 mg or less, about 1000 mg or less, about 700 mg or less, about 600 mg or less, about 1 mg to about 4,000 mg, about 1 mg to about 1,000 mg, about 10 mg to about 1000 mg, about 50 mg to about 1000 mg, about 10 mg to about 600 mg, about 40 mg to about 600 mg, about 50 mg to about 600 mg, or about 100 mg to about 600 mg, about 40 mg to about 2000 mg, about 40 mg to about 800 mg, about 50 mg to about 800 mg, or about 100 mg to about 800 mg, about 40 mg to about 1000 mg, about 50 mg to about 1000 mg, or about 100 mg to about 1000 mg, or any monthly dose in a range bounded by, or between, any of these values. In some embodiments, the monthly oral dose of zoledronic acid is less than about 1000 $mg/m^2$, less than about 800 $mg/m^2$, less than about 600 $mg/m^2$, about 10 $mg/m^2$ to about 1000 $mg/m^2$, about 50 $mg/m^2$ to about 800 $mg/m^2$, about 70 $mg/m^2$ to about 700 $mg/m^2$, about 100 $mg/m^2$ to about 700 $mg/m^2$, about 100 $mg/m^2$ to about 600 $mg/m^2$, about 50 $mg/m^2$ to about 200 $mg/m^2$, about 300 $mg/m^2$ to about 600 $mg/m^2$, about 450 $mg/m^2$ to about 600 $mg/m^2$, about 300 $mg/m^2$ to about 1000 $mg/m^2$, about 400 $mg/m^2$ to about 1000 $mg/m^2$, about 500 $mg/m^2$ to about 1000 $mg/m^2$, about 400 $mg/m^2$ to about 700 $mg/m^2$, about 500 $mg/m^2$ to about 600 $mg/m^2$, about 540 $mg/m^2$, or any amount of zoledronic acid in a range bounded by, or between, any of these values. A monthly dose may be given as a single dose, or as two or more individual doses administered during the month. In some embodiments, the monthly dose is administered in 2 or 3 weekly doses. In some embodiments, the monthly dose is administered in 4 or 5 weekly doses. In some embodiments, the monthly dose is administered in 28 to 31 daily doses. In some embodiments, the monthly dose is administered in 5 to 10 individual doses during the month. The monthly dose may be administered for only 1 month, or may be repeatedly administered for 2 or more months.

The oral zoledronic acid, or disodium salt thereof, may be administered in combination with about 0.1 mg to about 10 mg of zoledronic acid, or a salt thereof, administered parenterally, such as intravenously. In some embodiments, about 50 mg, about 100 mg, or about 150 mg of the disodium salt of zoledronic acid is administered orally in combination with 1 mg parenteral, such as intravenous, zoledronic acid. In some embodiments the parenteral dose of zoledronic acid is about 0.25 mg to about 25 mg, about 0.25 mg to about 10 mg, or about 0.5 mg to about 7.5 mg.

The oral bioavailability of zoledronic acid in a dosage form can vary. Some dosage forms may have ingredients added to enhance the bioavailability. However, bioavailability enhancement is not necessary for an oral dosage form to be effective. In some embodiments, the dosage form is substantially free of bioavailability-enhancing agents. In some embodiments, an oral dosage form may have an oral bioavailability of zoledronic acid of about 0.01% to about 10%, about 0.1% to about 7%, about 0.1% to about 5%, etc. Without ingredients or other methods to enhance bioavailability, zoledronic acid typically has a low bioavailability in an oral dosage form. In some embodiments, the oral bioavailability of zoledronic acid is unenhanced or substantially unenhanced. For example, the oral bioavailability of zoledronic acid can be about 0.01% to about 5%, about 0.01% to about 4%, about 0.1% to about 3%, about 0.1% to about 2%, about 0.2% to about 2%, about 0.2% to about 1.5%, about 0.3% to about 1.5%, about 0.3% to about 1%, about 0.1 % to about 0.5%, about 0.3% to about 0.5%, about 0.5% to about 1%, about 0.6% to about 0.7%, about 0.7% to about 0.8%, about 0.8% to about 0.9%, about 0.9%, about 1% to about 1.1%, about 1.1 % to about 1.2%, about 1.2% to about 1.3%, about 1.3% to about 1.4%, about 1.4% to about 1.5%, about 1.5% to about 1.6%, about 1.6% to about 1.8%, or about 1.8% to about 2%.

One embodiment is a pharmaceutical composition comprising zoledronic acid wherein the oral bioavailability of zoledronic acid in the dosage form is from about 0.01 % to about 10%.

In some embodiments, the oral bioavailability of zoledronic acid in the dosage form is about 0.01 % to about 5%.

In some embodiments, the oral bioavailability of zoledronic acid in the dosage form is about 0.1% to about 7%.

In some embodiments, the oral bioavailability of zoledronic acid in the dosage form is about 0.1% to about 5%.

In some embodiments, the oral bioavailability of zoledronic acid in the dosage form is about 0.1% to about 3%.

In some embodiments, the oral bioavailability of zoledronic acid in the dosage form is about 0.1% to about 2%.
In some embodiments, the oral bioavailability of zoledronic acid in the dosage form is about 0.2% to about 2%.
In some embodiments, the oral bioavailability of zoledronic acid in the dosage form is about 0.2% to about 1.5%.
In some embodiments, the oral bioavailability of zoledronic acid in the dosage form is about 0.3% to about 1.5%.
In some embodiments, the oral bioavailability of zoledronic acid in the dosage form is about 0.3% to about 1.0%.
In some embodiments, an oral dosage form comprises about 10 mg to about 300 mg of zoledronic acid, and is administered daily for about 2 to about 15 consecutive days. This regimen may be repeated once monthly, once every two months, once every three months, once every four months, once every five months, once every six months, once yearly, or once every two years.
In some embodiments, an oral dosage form comprises about 10 mg to about 150 mg or about 10 mg to about 100 mg of zoledronic acid, and is administered daily for about 2 to about 15 consecutive days. This regimen may be repeated once monthly, once every two months, once every three months, once every four months, once every five months, once every six months, once yearly, or once every two years.
In some embodiments, an oral dosage form comprises about 10 mg to about 150 mg or about 10 mg to about 100 mg of zoledronic acid, and is administered daily for about 5 to about 10 consecutive days. This regimen may be repeated once monthly, once every two months, once every three months, once every four months, once every five months, once every six months, once yearly, or once every two years.
In some embodiments, an oral dosage form comprises about 40 mg to about 150 mg of zoledronic acid, and is administered daily for about 5 to about 10 consecutive days. This regimen may be repeated once monthly, once every two months, once every three months, once every four months, once every five months, once every six months, once yearly, or once every two years.
In some embodiments, the oral zoledronic acid may be administered as one dose of about 100 mg to about 2000 mg. In some embodiments, the oral zoledronic acid may be administered as one dose of about 300 mg to about 1500 mg. In some embodiments, the oral zoledronic acid may be administered as one dose of about 200 mg to about 1000 mg. The dose of zoledronic acid may be administered in a single or divided dose.
Zoledronic acid may be formulated for oral administration, for example, with an inert diluent or with an edible carrier, or it may be enclosed in hard or soft shell gelatin capsules, compressed into tablets, or incorporated directly with the food of the diet. For oral therapeutic administration, the active compound may be incorporated with an excipient and used in the form of ingestible tablets, buccal tablets, coated tablets, troches, capsules, elixirs, dispersions, suspensions, solutions, syrups, wafers, patches, and the like.
Tablets, troches, pills, capsules and the like may also contain one or more of the following: a binder such as gum tragacanth, acacia, corn starch or gelatin; an excipient, such as dicalcium phosphate; a disintegrating agent such as corn starch, potato starch, alginic acid and the like; a lubricant such as magnesium stearate; a sweetening agent such as sucrose, lactose or saccharin; or a flavoring agent such as peppermint, oil of wintergreen or cherry flavoring. When the unit dosage form is a capsule, it may contain, in addition to materials of the above type, a liquid carrier. Various other materials may be present as coating, for instance, tablets, pills, or capsules may be coated with shellac, sugar or both. A syrup or elixir may contain the active compound, sucrose as a sweetening agent, methyl and propylparabens as preservatives, a dye and flavoring, such as cherry or orange flavor. It may be desirable for material in a dosage form or pharmaceutical composition to be pharmaceutically pure and substantially non toxic in the amounts employed.
Some compositions or dosage forms may be a liquid, or may comprise a solid phase dispersed in a liquid.
Zoledronic acid may be formulated for parental or intraperitoneal administration. Solutions of the active compounds as free acids or pharmacologically acceptable salts can be prepared in water suitably mixed with a surfactant, such as hydroxypropylcellulose. A dispersion can also have an oil dispersed within, or dispersed in, glycerol, liquid polyethylene glycols, and mixtures thereof. Under ordinary conditions of storage and use, these preparations may contain a preservative to prevent the growth of microorganisms.
In some embodiments, an oral dosage form may comprise a silicified microcrystalline cellulose such as Prosolv. For example, about 20% (wt/wt) to about 70% (wt/wt), about 10% (wt/wt) to about 20% (wt/wt), about 20% (wt/wt) to about 40% (wt/wt), about 25% (wt/wt) to about 30% (wt/wt), about 40% (wt/wt) to about 50% (wt/wt), or about 45% (wt/wt) to about 50% (wt/wt) silicified microcrystalline cellulose may be present in an oral dosage form or a unit of an oral dosage form.
In some embodiments, an oral dosage form may comprise a crosslinked polyvinylpyrrolidone such as crospovidone. For example, about 1% (wt/wt) to about 10% (wt/wt), about 1% (wt/wt) to about 5% (wt/wt), or about 1% (wt/wt) to about 3% (wt/wt) crosslinked polyvinylpyrrolidone may be present in an oral dosage form or a unit of an oral dosage form.
In some embodiments, an oral dosage form may comprise a fumed silica such as Aerosil. For example, about 0.1% (wt/wt) to about 10% (wt/wt), about 0.1% (wt/wt) to about 1% (wt/wt), or about 0.4% (wt/wt) to about 0.6% (wt/wt) fumed silica may be present in an oral dosage form or a unit of an oral dosage form.
In some embodiments, an oral dosage form may comprise magnesium stearate. For example, about 0.1% (wt/wt) to about 10% (wt/wt), about 0.1 % (wt/wt) to about 1% (wt/wt), or about 0.4% (wt/wt) to about 0.6% (wt/wt)

magnesium stearate may be present in an oral dosage form or a unit of an oral dosage form.

An oral dosage form comprising zoledronic acid or another bisphosphonate may be included in a pharmaceutical product comprising more than one unit of the oral dosage form.

A pharmaceutical product containing oral dosage forms for daily use can contain 28, 29, 30, or 31 units of the oral dosage form for a monthly supply. An approximately 6 week daily supply can contain 40 to 45 units of the oral dosage form. An approximately 3 month daily supply can contain 85 to 95 units of the oral dosage form. An approximately six-month daily supply can contain 170 to 200 units of the oral dosage form. An approximately one year daily supply can contain 350 to 380 units of the oral dosage form.

A pharmaceutical product containing oral dosage forms for weekly use can contain 4 or 5 units of the oral dosage form for a monthly supply. An approximately 2 month weekly supply can contain 8 or 9 units of the oral dosage form. An approximately 6 week weekly supply can contain about 6 units of the oral dosage form. An approximately 3 month weekly supply can contain 12, 13 or 14 units of the oral dosage form. An approximately six-month weekly supply can contain 22 to 30 units of the oral dosage form. An approximately one year weekly supply can contain 45 to 60 units of the oral dosage form.

A pharmaceutical product may accommodate other dosing regimes. For example, a pharmaceutical product may comprise 5 to 10 units of the oral dosage form, wherein each unit of the oral dosage form contains about 40 mg to about 150 mg of zoledronic acid. Some pharmaceutical products may comprise 1 to 10 units of the oral dosage form, wherein the product contains about 200 mg to about 2000 mg of zoledronic acid. For such a product, each unit of the oral dosage form may be taken daily for 1 to 10 days or 5 to 10 days during a month, such as at the beginning of a month.

Some oral dosage forms comprising zoledronic acid or a salt thereof may have enteric coatings.

In the examples below, zoledronic acid was administered in the disodium salt form as disodium zoledronate tetrahydrate. No bioavailability enhancing agents were used in the test compositions.

**Example 1**

**Effect of Orally Administered Zoledronic Acid in Rat Model of Inflammatory Pain**

**Method:**

The effect of orally administered zoledronic acid on inflammatory pain was examined using the rat complete Freund's adjuvant (CFA) model. Inflammatory pain was induced by injection of 100% CFA in a 75 μL volume into the left hind paws of Sprague-Dawley rats on day 0, followed by assessments on days 1-3. Animals were orally administered vehicle (control), zoledronic acid 18 mg/m$^2$ (or 3 mg/kg), zoledronic acid 120 mg/m$^2$ (or 20 mg/kg), or zoledronic acid 900 mg/m$^2$ (or 150 mg/kg) daily on days 1-3. Drug was dissolved in distilled water and prepared fresh daily. Animals were fasted prior to dosing. Under current FDA guidelines for extrapolating starting dosages from animals to humans, dosages expressed in mg/m$^2$ are considered equivalent between mammalian species. Thus, for example, 18 mg/m$^2$ in a rat is considered equivalent to 18 mg/m$^2$ in a human being, while 3 mg/kg in a rat may not be equivalent to 3 mg/kg in a human being.

Values for inflammatory pain (mechanical hyperalgesia) in the vehicle and drug-treated animals were obtained on day 0 prior to CFA injection, and at baseline and post-treatment on days 1-3. Pain was assessed using a digital Randall-Selitto device (dRS; IITC Life Sciences, Woodland Hills, CA). Animals were placed in a restraint sling that suspended the animal, leaving the hind limbs available for testing. Paw compression threshold was measured by applying increasing pressure to the plantar surface of the hind paw with a dome-shaped tip placed between the 3rd and 4th metatarsus. Pressure was applied gradually over approximately 10 seconds. Measurements were taken from the first observed nocifensive behavior of vocalization, struggle or withdrawal. A cut-off value of 300 g was used to prevent injury to the animal.

Reversal of inflammatory pain was calculated according to the formula:

% reversal = ( Post-treatment – Post-CFA baseline)/(Pre-CFA baseline – Post-CFA baseline) x 100.

The experiment was carried out using 9-10 animals per group. Results:

Oral administration of zoledronic acid significantly improved inflammatory pain thresholds compared to vehicle. Pain threshold measurements taken at various times are shown in FIG. 1. Paw compression thresholds in the 18 mg/m$^2$ group were higher than for vehicle during the entire measurement period after 30 minutes from the start of treatment. On day three, paw compression thresholds for both the 18 mg/m$^2$ and 900 mg/m$^2$ groups were greater than for vehicle.

An improvement in pain threshold of 49% and 83% from baseline was observed for the 18 mg/$m^2$ and the 900 mg/$m^2$ groups respectively.

Orally administered zoledronic acid produced a 29% reversal of inflammatory pain at the 18 mg/$m^2$, and a 48% reversal at the 900 mg/$m^2$ dose. This magnitude of effect is comparable to that obtained with clinical doses of commercially available NSAIDs when tested in a similar model of inflammatory pain. Under current FDA guidelines, the reference body surface area of a human adult is 1.62 $m^2$. Thus, a daily dose of 18 mg/$m^2$ corresponds to a monthly dose of about 500-560 mg/$m^2$ or a human dose of about 800-900 mg.

Surprisingly, the two higher doses resulted in thresholds that were lower than vehicle on the first two days of dosing. The 120 mg/$m^2$ group was approximately equal or inferior to vehicle at all time points during the assessment period. While the 900 mg/$m^2$ group showed effectiveness on day 3, this result was accompanied by significant toxicity necessitating euthanization of all the animals in this group two days after cessation of dosing.

**Example 2**

**Effect of Orally Administered Zoledronic Acid in Rat Model of Arthritis Pain**

**Method:**

The effect of orally administered zoledronic acid on arthritis pain was examined in the rat complete Freund's adjuvant (CFA) model of arthritis pain. In this model, injection of 100% complete Freund's adjuvant (CFA) in a 75 μL volume into the left hind paws is followed by a 10-14 day period to allow for the development of arthritis pain. Animals were orally administered vehicle (control), zoledronic acid 54 mg/$m^2$ (or 9 mg/kg), or zoledronic acid 360 mg/$m^2$ (or 60 mg/kg), divided in three equal daily doses on the first three days post CFA injection. Drug was dissolved in distilled water and prepared fresh daily. Animals were fasted prior to dosing.

Arthritis pain (mechanical hyperalgesia) in the vehicle and drug-treated animals was evaluated on day 14 post CFA injection using a digital Randall-Selitto device (dRS; IITC Life Sciences, Woodland Hills, CA). Animals were placed in a restraint sling that suspended the animal, leaving the hind limbs available for testing. Paw compression threshold was measured by applying increasing pressure to the plantar surface of the hind paw with a dome-shaped tip placed between the 3rd and 4th metatarsus. Pressure was applied gradually over approximately 10 seconds. Measurements were taken from the first observed nocifensive behavior of vocalization, struggle or withdrawal. A cut-off value of 300 g was used to prevent injury to the animal.

Reversal of arthritis pain in the ipsilateral (CFA-injected) paw was calculated according to the formula:

% reversal = (ipsilateral drug threshold – ipsilateral vehicle threshold)/(contralateral vehicle threshold – ipsilateral vehicle threshold) x 100.

The experiment was carried out using 7-10 animals per group. Results:

Oral administration of zoledronic acid significantly improved arthritis pain thresholds compared to vehicle. As shown in FIGS. 2A and 2B, orally administered zoledronic acid produced a dose-dependent reversal of arthritis pain. A reversal of 33% was observed in the 54 mg/$m^2$ group, and reversal of 54% was observed in the 360 mg/$m^2$ group. Under current FDA guidelines, the reference body surface area of a human adult is 1.62 $m^2$. Thus, 54 mg/$m^2$ in a rat is equivalent to an implied human dose of about 87 mg, and 360 mg/$m^2$ in a rat is equivalent to an implied human dose of about 583 mg.

**Example 3. Treatment of Complex Regional Pain Syndrome with Orally Administered Zoledronic Acid.**

The effect of orally administered zoledronic acid was examined in the rat tibia fracture model of complex regional pain syndrome (CRPS). CRPS was induced in the rats by fracturing the right distal tibias of the animals and casting the fractured hindpaws for 4 weeks, as described in Guo TZ et al. (Pain. 2004;108:95-107). This animal model has been shown to replicate the inciting trauma, natural history, signs, symptoms, and pathologic changes observed in human CRPS patients (Kingery WS et al., Pain. 2003;104:75-84).

Animals were orally administered either vehicle (control) or zoledronic acid, in a dosage of 18 mg/$m^2$/day (3 mg/kg/day) for 28 days, starting on the day of fracture and casting. Drug was dissolved in distilled water and administered by gavage. Animals were fasted for 4 hours before and 2 hours after dosing. At the end of the 28-day period, casts were removed, and on the following day, the rats were tested for hindpaw pain, edema, and warmth.

## Pain assessments

Pain was assessed by measuring hyperalgesia, and weight bearing.

To measure hyperalgesia, an up-down von Frey testing paradigm was used. Rats were placed in a clear plastic cylinder (20 cm in diameter) with a wire mesh bottom and allowed to acclimate for 15 minutes. The paw was tested with one of a series of eight von Frey hairs ranging in stiffness from 0.41 g to 15.14 g. The von Frey hair was applied against the hindpaw plantar skin at approximately midsole, taking care to avoid the tori pads. The fiber was pushed until it slightly bowed and then it was jiggled in that position for 6 seconds. Stimuli were presented at an interval of several seconds. Hindpaw withdrawal from the fiber was considered a positive response. The initial fiber presentation was 2.1 g and the fibers were presented according to the up-down method of Dixon to generate six responses in the immediate vicinity of the 50% threshold. Stimuli were presented at an interval of several seconds.

An incapacitance device (IITC Inc. Life Science, Woodland, CA, USA) was used to measure hindpaw weight bearing, a postural effect of pain. The rats were manually held in a vertical position over the apparatus with the hindpaws resting on separate metal scale plates and the entire weight of the rat was supported on the hindpaws. The duration of each measurement was 6 seconds and 10 consecutive measurements were taken at 60-second intervals. Eight readings (excluding the highest and lowest ones) were averaged to calculate the bilateral hindpaw weight-bearing values. Weight bearing data were analyzed as the ratio between right (fracture) and left hindpaw weight bearing values ((2R/(R+L)) ×100%).

## Edema assessment

A laser sensor technique was used to determine the dorsal-ventral thickness of the hindpaw. Before baseline testing the bilateral hindpaws were tattooed with a 2 to 3 mm spot on the dorsal skin over the midpoint of the third metatarsal. For laser measurements each rat was briefly anesthetized with isoflurane and then held vertically so the hindpaw rested on a table top below the laser. The paw was gently held flat on the table with a small metal rod applied to the top of the ankle joint. Using optical triangulation, a laser with a distance measuring sensor was used to determine the distance to the table top and to the top of the hindpaw at the tattoo site and the difference was used to calculate the dorsal-ventral paw thickness. The measurement sensor device used in these experiments (4381 Precicura, Limab, Goteborg, Sweden) has a measurement range of 200 mm with a 0.01 mm resolution.

## Hindpaw temperature measurement

The temperature of the hindpaw was measured using a fine wire thermocouple (Omega, Stanford, CT, USA) applied to the paw skin. Six sites were tested per hindpaw. The six measurements for each hindpaw were averaged for the mean temperature.

## Results

As illustrated in FIG. 3, treatment with orally administered zoledronic acid reversed pain, restored weight bearing, and prevented edema as compared to vehicle treated animals.

As illustrated in FIG. 4, von Frey pain thresholds for the right (fracture) hindpaw were reduced by 72% versus the contralateral (normal) hindpaw in vehicle treated animals. Zoledronate treatment reversed fracture induced pain by 77% as compared to vehicle treatment.

As illustrated in FIG. 5, reduction in weight bearing, a postural effect of pain, was significantly higher in the vehicle treated group as compared to the zoledronic acid treated group. Weight bearing on the fracture hindlimb was reduced to 55% of normal in the vehicle treated group. Zoledronate treatment significantly restored hindlimb weight bearing as compared to vehicle treatment (86% of normal).

As illustrated in FIG. 6, the expected increase in hindpaw thickness was greater in the vehicle treated group as compared to the zoledronic acid treated group, reflecting the development of edema. Zoledronate treatment reduced hindpaw edema by 60% versus vehicle treatment.

Zoledronic acid reduced hindpaw warmth by 5% versus vehicle treatment.

The daily dose in the above experiment was 18 $mg/m^2$/day. Under current FDA guidelines, the reference body surface area of a human adult is 1.62 $m^2$. Thus, a daily dose of 18 $mg/m^2$ corresponds to a monthly dose of about 500-560 $mg/m^2$ or a human dose of about 800-900 mg.

## Example 6. Solubility of Disodium Salt of Zoledronic Acid

The aqueous solubility of zoledronic acid and disodium zoledronate tetrahydrate was determined. One gram

of the test compound was measured in to a beaker. Demineralized water (pH 5.5) was then added in small increments to the test compound, and sonification was applied to the mixture. The procedure was continued until complete dissolution was achieved. Full dissolution was determined to have been reached when a clear solution was present with no visible material. The volume of water required to reach full dissolution was used to calculate a solubility value expressed in grams per 100 mL. The procedure was performed for each compound.

**Results**

As shown in FIG. 7, the aqueous solubility of disodium zoledronate tetrahydrate is approximately 50 times that of zoledronic acid. Disodium zoledronate tetrahydrate has a solubility of 12.5 g/100 mL compared to only 0.25 g/100 mL for zoledronic acid.

Unless otherwise indicated, all numbers expressing quantities of ingredients, properties such as molecular weight, reaction conditions, and so forth used in the specification and claims are to be understood in all instances as indicating both the exact values as shown and as being modified by the term "about." Accordingly, unless indicated to the contrary, the numerical parameters set forth in the specification and attached claims are approximations that may vary depending upon the desired properties sought to be obtained. At the very least, and not as an attempt to limit the application of the doctrine of equivalents to the scope of the claims, each numerical parameter should at least be construed in light of the number of reported significant digits and by applying ordinary rounding techniques.

The terms "a," "an," "the" and similar referents used in the context of describing the invention (especially in the context of the following claims) are to be construed to cover both the singular and the plural, unless otherwise indicated herein or clearly contradicted by context. All methods described herein can be performed in any suitable order unless otherwise indicated herein or otherwise clearly contradicted by context. The use of any and all examples, or exemplary language (e.g., "such as") provided herein is intended merely to better illuminate the invention and does not pose a limitation on the scope of any claim. No language in the specification should be construed as indicating any non-claimed element essential to the practice of the invention.

Groupings of alternative elements or embodiments disclosed herein are not to be construed as limitations. Each group member may be referred to and claimed individually or in any combination with other members of the group or other elements found herein. It is anticipated that one or more members of a group may be included in, or deleted from, a group for reasons of convenience and/or patentability. When any such inclusion or deletion occurs, the specification is deemed to contain the group as modified thus fulfilling the written description of all Markush groups used in the appended claims.

Certain embodiments are described herein, including the best mode known to the inventors for carrying out the invention. Of course, variations on these described embodiments will become apparent to those of ordinary skill in the art upon reading the foregoing description. The inventor expects skilled artisans to employ such variations as appropriate, and the inventors intend for the invention to be practiced otherwise than specifically described herein. Accordingly, the claims include all modifications and equivalents of the subject matter recited in the claims as permitted by applicable law. Moreover, any combination of the above-described elements in all possible variations thereof is contemplated unless otherwise indicated herein or otherwise clearly contradicted by context.

In closing, it is to be understood that the embodiments disclosed herein are illustrative of the principles of the claims. Other modifications that may be employed are within the scope of the claims. Thus, by way of example, but not of limitation, alternative embodiments may be utilized in accordance with the teachings herein. Accordingly, the claims are not limited to embodiments precisely as shown and described.

The following embodiments are specifically contemplated:

**Embodiment 1.** A method of relieving inflammatory pain comprising administering an oral dosage form containing zoledronic acid to a mammal in need thereof, wherein the mammal receives a total monthly dose of zoledronic acid that is about 800 $mg/m^2$ or less based upon the body surface area of the mammal.

**Embodiment 2.** The method of embodiment 1, wherein the mammal is a human being that receives a total monthly dose of zoledronic acid that is about 30 $mg/m^2$ to about 700 $mg/m^2$.

**Embodiment 3.** The method of embodiment 2, wherein the total monthly dose is administered in 4 or 5 weekly doses.

**Embodiment 4.** The method of embodiment 2, wherein the total monthly dose is administered in 28 to 31 daily doses.

**Embodiment 5.** The method of embodiment 2, wherein the total monthly dose is administered in 5 to 10 individual doses during the month.

**Embodiment 6.** The method of embodiment 1, wherein the mammal is a human being that receives a total weekly dose of zoledronic acid that is about 10 mg to about 300 mg.

**Embodiment 7.** The method of embodiment 6, wherein the total weekly dose is a single dose, administered once a week.

**Embodiment 8.** The method of embodiment 6, wherein the total weekly dose is administered in 2 to 7 individual

doses during the week.

**Embodiment 9.** The method of embodiment 1, wherein the mammal is a human being that receives a total weekly dose of zoledronic acid that is about 10 mg to about 150 mg.

**Embodiment 10.** The method of any preceding embodiment, wherein the mammal experiences significant pain relief more than 3 hours after administration of the dosage form.

**Embodiment 11.** The method of embodiment 10, wherein the mammal experiences significant pain relief during at least a part of a time from about 3 hours to about 24 hours after administration of the dosage form.

**Embodiment 12.** The method of embodiment 10, wherein the mammal experiences significant pain relief during at least a part of a time from about 3 hours to about 3 weeks after administration of the dosage form.

**Embodiment 13.** A method of relieving inflammatory pain comprising administering an oral dosage form containing zoledronic acid to a mammal in need thereof, wherein the oral dosage form contains about 10 $mg/m^2$ to about 20 $mg/m^2$ of zoledronic acid based upon the body surface area of the mammal.

**Embodiment 14.** The method of embodiment 13, wherein the oral dosage form contains about 15 $mg/m^2$ to about 20 $mg/m^2$ of zoledronic acid based upon the body surface area of the mammal.

**Embodiment 15.** A method of relieving inflammatory pain comprising orally administering to a mammal in need thereof, about 300 $mg/m^2$ to about 600 $mg/m^2$ of zoledronic acid per month to the mammal, based upon the body surface area of the mammal.

**Embodiment 16.** The method of embodiment 15, comprising orally administering about 450 $mg/m^2$ to about 600 $mg/m^2$ of zoledronic acid per month to the mammal, based upon the body surface area of the mammal.

**Embodiment 17.** The method of any preceding embodiment, wherein the mammal is not suffering from bone metastasis.

**Embodiment 18.** The method of any preceding embodiment, wherein the mammal is not suffering from cancer.

**Embodiment 19.** The method of any preceding embodiment, wherein the zoledronic acid is administered as a salt of a dianion of zoledronic acid.

**Embodiment 20.** A method of relieving pain associated with an arthritis comprising administering an oral dosage form containing zoledronic acid to a human being in need thereof.

**Embodiment 21.** The method of embodiment 20, wherein the human being receives a total monthly dose of zoledronic acid that is about 40 mg to about 2000 mg.

**Embodiment 2**2. The method of embodiment 21, wherein the total monthly dose is administered in 4 or 5 weekly doses.

**Embodiment 23.** The method of embodiment 21, wherein the total monthly dose is administered in 28 to 31 daily doses.

**Embodiment 24.** The method of embodiment 21, wherein the total monthly dose is administered in 5 to 10 individual doses during the month.

**Embodiment 25.** The method of embodiment 20, wherein the human being receives a total weekly dose of zoledronic acid that is about 100 mg to about 300 mg.

**Embodiment 2**6. The method of embodiment 25, wherein the total weekly dose is a single dose, administered once a week.

**Embodiment 27.** The method of embodiment 25, wherein the total weekly dose is administered in 2 to 7 individual doses during the week.

**Embodiment 28.** The method of embodiment 20, wherein the human being receives a total weekly dose of zoledronic acid that is about 10 mg to about 100 mg.

**Embodiment 29.** The method of any of embodiments 20-28, wherein the human being experiences significant pain relief more than 3 hours after administration of the dosage form.

**Embodiment 30.** The method of embodiment 29, wherein the human being experiences significant pain relief during at least a part of a time from about 3 hours to about 24 hours after administration of the dosage form.

**Embodiment 31.** The method of embodiment 29, wherein the human being experiences significant pain relief during at least a part of a time from about 3 hours to about 3 weeks after administration of the dosage form.

**Embodiment 32.** The method of any of embodiments 20-31, wherein the dosage form contains about 10 $mg/m^2$ to about 20 $mg/m^2$ of zoledronic acid based upon the body surface area of the human being.

**Embodiment 33.** The method of embodiment 32, wherein the dosage form contains about 15 $mg/m^2$ to about 20 $mg/m^2$ of zoledronic acid based upon the body surface area of the human being.

**Embodiment 34.** The method of any of embodiments 20-33, wherein about 50 $mg/m^2$ to about 200 $mg/m^2$ of zoledronic acid is orally administered per month, based upon the body surface area of the human being.

**Embodiment 35.** The method of any of embodiments 20-31, wherein the dosage form contains about 80 $mg/m^2$ to about 150 $mg/m^2$ of zoledronic acid based upon the body surface area of the human being.

**Embodiment 36.** The method of embodiment 35, wherein about 300 $mg/m^2$ to about 1000 $mg/m^2$ of zoledronic acid is orally administered per month, based upon the body surface area of the human being.

**Embodiment 37.** The method of any of embodiments 20-36, wherein the human being is not suffering from bone metastasis.

**Embodiment 38.** The method of any of embodiments 20-37, wherein the human being is not suffering from cancer.

**Embodiment 39.** The method of any preceding embodiment, wherein the zoledronic acid is in the disodium salt form.

**Embodiment 40.** An oral dosage form comprising zoledronic acid, wherein the oral bioavailability of zoledronic acid in the dosage form is about 0.01% to about 4%.

**Embodiment 41.** The oral dosage form of embodiment 40, wherein the oral dosage form contains about 10 mg to about 300 mg of zoledronic acid.

**Embodiment 42.** The oral dosage form of embodiment 40, wherein the oral dosage form contains about 10 mg to about 50 mg of zoledronic acid.

**Embodiment 43.** The oral dosage form of any of embodiments 40-42, wherein the oral bioavailability of zoledronic acid in the dosage form is about 0.1 % to about 2%.

**Embodiment 44.** A pharmaceutical product comprising more than one unit of an oral dosage form of embodiment 40.

**Embodiment 45.** The pharmaceutical product of embodiment 44, wherein each unit of the oral dosage form contains about 1 mg to about 50 mg of zoledronic acid.

**Embodiment 46.** The pharmaceutical product of embodiment 45, comprising 28, 29, 30, or 31 units of the oral dosage form, for a total of about 28 mg to about 1600 mg of zoledronic acid to be administered in about 1 month.

**Embodiment 47.** The pharmaceutical product of embodiment 45, comprising 85 to 95 units of the oral dosage form, for a total of about 85 mg to about 4800 mg of zoledronic acid to be administered in about 3 months.

**Embodiment 48.** The pharmaceutical product of embodiment 45, comprising 170 to 200 units of the oral dosage form, for a total of about 170 mg to about 10,000 mg of zoledronic acid to be administered in about 6 months.

**Embodiment 49.** The pharmaceutical product of embodiment 45, comprising 350 to 380 units of the oral dosage form, for a total of about 350 mg to about 19,000 mg of zoledronic acid to be administered in about 1 year.

**Embodiment 50.** The pharmaceutical product of embodiment 44, wherein each unit of the oral dosage form contains about 10 mg to about 300 mg.

**E**mbodiment **51.** The pharmaceutical product of embodiment 50, comprising 4 or 5 units of the oral dosage form, for a total of about 40 mg to about 1500 mg of zoledronic acid to be administered within a period of about 1 month.

**Embodiment 52.** The pharmaceutical product of embodiment 50, comprising 8 or 9 units of the oral dosage form, for a total of about 80 mg to about 2700 mg of zoledronic acid to be administered in about 2 months.

**Embodiment 53.** The pharmaceutical product of embodiment 50, comprising 12, 13 or 14 units of the oral dosage form, for a total of about 120 mg to about 4200 mg of zoledronic acid to be administered in about 3 months.

**Embodiment 54.** The pharmaceutical product of embodiment 50, comprising 22 to 30 units of the oral dosage form, for a total of about 220 mg to about 9000 mg of zoledronic acid to be administered in about 6 months.

**Embodiment 55.** The pharmaceutical product of embodiment 50, comprising 45 to 60 units of the oral dosage form, for a total of about 450 mg to about 18000 mg of zoledronic acid to be administered in about 1 year.

**Embodiment 56.** The pharmaceutical product of embodiment 44, comprising 1 to 10 units of the oral dosage form, wherein the product contains about 200 mg to about 2000 mg of zoledronic acid.

**Embodiment 57.** The oral dosage form of any preceding embodiment, wherein the zoledronic acid is in the form of a sodium salt.

**Embodiment 58.** The oral dosage form of any preceding embodiment, wherein the zoledronic acid is in a form that has an aqueous solubility greater than 1% (w/v).

**Embodiment 59.** The oral dosage form of any preceding embodiment, wherein the zoledronic acid is in a form that has an aqueous solubility of about 5% (w/v) to about 50% (w/v).

**Embodiment 60.** An oral dosage form comprising zoledronic acid and an excipient, wherein the zoledronic acid is in a form that has an aqueous solubility greater than 1% (w/v).

**Embodiment 61.** The oral dosage form of embodiment 60, wherein the zoledronic acid is in a form that has an aqueus solubility of about 5% (w/v) to about 50% (w/v).

**Embodiment 62.** A method of treating complex regional pain syndrome comprising administering an oral dosage form containing zoledronic acid to a mammal in need thereof.

**Embodiment 63.** The method of embodiment 62, wherein the mammal is a human being that receives an amount of zoledronic acid that is about 30 $mg/m^2$ to about 700 $mg/m^2$ in a period of one month or less.

**Embodiment 64.** The method of embodiment 63, wherein 4 or 5 weekly doses are administered in a period of one month or less.

**Embodiment 65.** The method of embodiment 63, wherein 28 to 31 daily doses are administered in a period of one month or less.

**Embodiment 66.** The method of embodiment 63, wherein 5 to 10 individual doses are administered during a period of one month or less.

**Embodiment 67.** The method of embodiment 63, wherein about 30 $mg/m^2$ to about 700 $mg/m^2$ of zoledronic acid

is administered during only one month.

**Embodiment 68.** The method of embodiment 63, wherein about 30 mg/$m^2$ to about 700 mg/$m^2$ of zoledronic acid is administered in a period of one month or less for 2 or more consecutive months.

**Embodiment 69.** The method of embodiment 62, wherein the mammal receives about 10 mg/$m^2$ to about 30 mg/$m^2$ of zoledronic acid daily.

**Embodiment 70.** The method of embodiment 62, wherein the mammal is a human being that receives a total weekly dose of zoledronic acid that is about 10 mg to about 300 mg.

**Embodiment 71.** The method of embodiment 70, wherein the total weekly dose is a single dose, administered once a week.

**Embodiment 72.** The method of embodiment 70, wherein the total weekly dose is administered in 2 to 7 individual doses during the week.

**Embodiment 73.** The method of any of embodiments 62-72, wherein the complex regional pain syndrome is complex regional pain syndrome type I.

**Embodiment 74.** The method of any of embodiments 62-72, wherein the complex regional pain syndrome is complex regional pain syndrome type II.

**Embodiment 75.** The method of any preceding embodiment, wherein the zoledronic acid is in a salt form.

**Embodiment 76.** The method of any of embodiments 62-75, wherein the dosage form contains about 10 mg/$m^2$ to about 20 mg/$m^2$ of zoledronic acid based upon the body surface area of the mammal.

**Embodiment 77.** The method of embodiment 76, wherein the dosage form contains about 15 mg/$m^2$ to about 20 mg/$m^2$ of zoledronic acid based upon the body surface area of the mammal.

**Embodiment 78.** A method of treating complex regional pain syndrome, comprising administering pamidronic acid to a human being in need thereof.

**Embodiment 79.** A method of treating complex regional pain syndrome, comprising administering neridronic acid to a human being in need thereof.

**Embodiment 80.** A method of treating complex regional pain syndrome, comprising administering olpadronic acid to a human being in need thereof.

**Embodiment 81.** A method of treating complex regional pain syndrome, comprising administering alendronic acid to a human being in need thereof.

**Embodiment 82.** A method of treating complex regional pain syndrome, comprising administering incadronic acid to a human being in need thereof.

**Embodiment 83.** A method of treating complex regional pain syndrome, comprising administering ibandronic acid to a human being in need thereof.

**Embodiment 84.** A method of treating complex regional pain syndrome, comprising administering risedronic acid to a human being in need thereof.

**Embodiment 85.** A method of treating pain, comprising administering pamidronic acid to a human being in need thereof.

**Embodiment 86.** A method of treating pain, comprising administering neridronic acid to a human being in need thereof.

**Embodiment 87.** A method of treating pain, comprising administering olpadronic acid to a human being in need thereof.

**Embodiment 88.** A method of treating pain, comprising administering alendronic acid to a human being in need thereof.

**Embodiment 89.** A method of treating pain, comprising administering incadronic acid to a human being in need thereof.

**Embodiment 90.** A method of treating pain, comprising administering ibandronic acid to a human being in need thereof.

**Embodiment 91.** A method of treating pain, comprising administering risedronic acid to a human being in need thereof.

**Embodiment 92.** A method of treating arthritis pain, comprising administering pamidronic acid to a human being in need thereof.

**Embodiment 93.** A method of treating arthritis pain, comprising administering neridronic acid to a human being in need thereof.

**Embodiment 94.** A method of treating arthritis pain, comprising administering olpadronic acid to a human being in need thereof.

**Embodiment 95.** A method of treating arthritis pain, comprising administering alendronic acid to a human being in need thereof.

**Embodiment 96.** A method of treating arthritis pain, comprising administering incadronic acid to a human being in need thereof.

**Embodiment 97.** A method of treating arthritis pain, comprising administering ibandronic acid to a human being in need thereof.

**Embodiment 98.** A method of treating arthritis pain, comprising administering risedronic acid to a human being in need thereof.

**Embodiment 99.** A method of treating inflammatory pain, comprising administering pamidronic acid to a human being in need thereof.

**Embodiment 100.** A method of treating inflammatory pain, comprising administering neridronic acid to a human being in need thereof.

**Embodiment 101.** A method of treating inflammatory pain, comprising administering olpadronic acid to a human being in need thereof.

**Embodiment 102.** A method of treating inflammatory pain, comprising administering alendronic acid to a human being in need thereof.

**Embodiment 103.** A method of treating inflammatory pain, comprising administering incadronic acid to a human being in need thereof.

**Embodiment 104.** A method of treating inflammatory pain, comprising administering ibandronic acid to a human being in need thereof.

**Embodiment 105.** A method of treating inflammatory pain, comprising administering risedronic acid to a human being in need thereof.

**Embodiment 106.** A method of treating complex regional pain syndrome, comprising administering etidronic acid to a human being in need thereof.

**Embodiment 107.** A method of treating pain, comprising administering etidronic acid to a human being in need thereof.

**Embodiment 108.** A method of treating arthritis pain, comprising administering etidronic acid to a human being in need thereof.

**Embodiment 109.** A method of treating inflammatory pain, comprising administering etidronic acid to a human being in need thereof.

**Embodiment 110.** A method of treating complex regional pain syndrome, comprising administering clodronic acid to a human being in need thereof.

**Embodiment 111.** A method of treating pain, comprising administering clodronic acid to a human being in need thereof.

**Embodiment 112.** A method of treating arthritis pain, comprising administering clodronic acid to a human being in need thereof.

**Embodiment 113.** A method of treating inflammatory pain, comprising administering clodronic acid to a human being in need thereof.

**Embodiment 114.** A method of treating complex regional pain syndrome, comprising administering tiludronic acid to a human being in need thereof.

**Embodiment 115.** A method of treating pain, comprising administering tiludronic acid to a human being in need thereof.

**Embodiment 116.** A method of treating arthritis pain, comprising administering tiludronic acid to a human being in need thereof.

**Embodiment 117.** A method of treating inflammatory pain, comprising administering tiludronic acid to a human being in need thereof.

**Embodiment 118.** The method of any of embodiments 78-117, wherein the active compound is orally administered.

**Embodiment 119.** The method of any of embodiments 78-117, wherein the active compound is parenterally administered.

## Claims

1. Zoledronic acid for use in a method of relieving pain in a mammal, wherein an oral dosage form comprising the disodium salt of zoledronic acid is administered to the mammal.

2. Zoledronic acid for the use according to claim 1, wherein the mammal is a human being and the pain is associated with complex regional pain syndrome.

3. Zoledronic acid for the use according to claim 1, wherein the mammal is a human being and the pain is associated with arthritis.

4. Zoledronic acid for the use according to any one of claims 1 to 3, wherein the disodium salt of zoledronic acid is administered in an amount about 800 $mg/m^2$ or less based upon the body surface area of the mammal.

5. Zoledronic acid for the use according to claim 4, wherein the mammal is a human being and wherein the total monthly dose is about 30 $mg/m^2$ to about 700 $mg/m^2$ based upon the body surface area of the mammal.

6. Zoledronic acid for use according to claim 1, wherein the mammal is a human being that receives a total weekly dose of zoledronic acid that is about 10 mg to about 300 mg.

7. Zoledronic acid for use according to claim 6, wherein the total weekly dose is a single dose, administered once a week.

8. Zoledronic acid for use according to claim 6 or claim 7, wherein the total weekly dose of zoledronic acid is about 10 mg to about 150 mg.

9. Zoledronic acid for use according to claim 1, wherein the oral dosage form contains about 10 $mg/m^2$ to about 20 $mg/m^2$ of zoledronic acid based upon the body surface area of the mammal.

10. Zoledronic acid for use according to claim 9, wherein the oral dosage form contains about 15 $mg/m^2$ to about 20 $mg/m^2$ of zoledronic acid based upon the body surface area of the mammal.

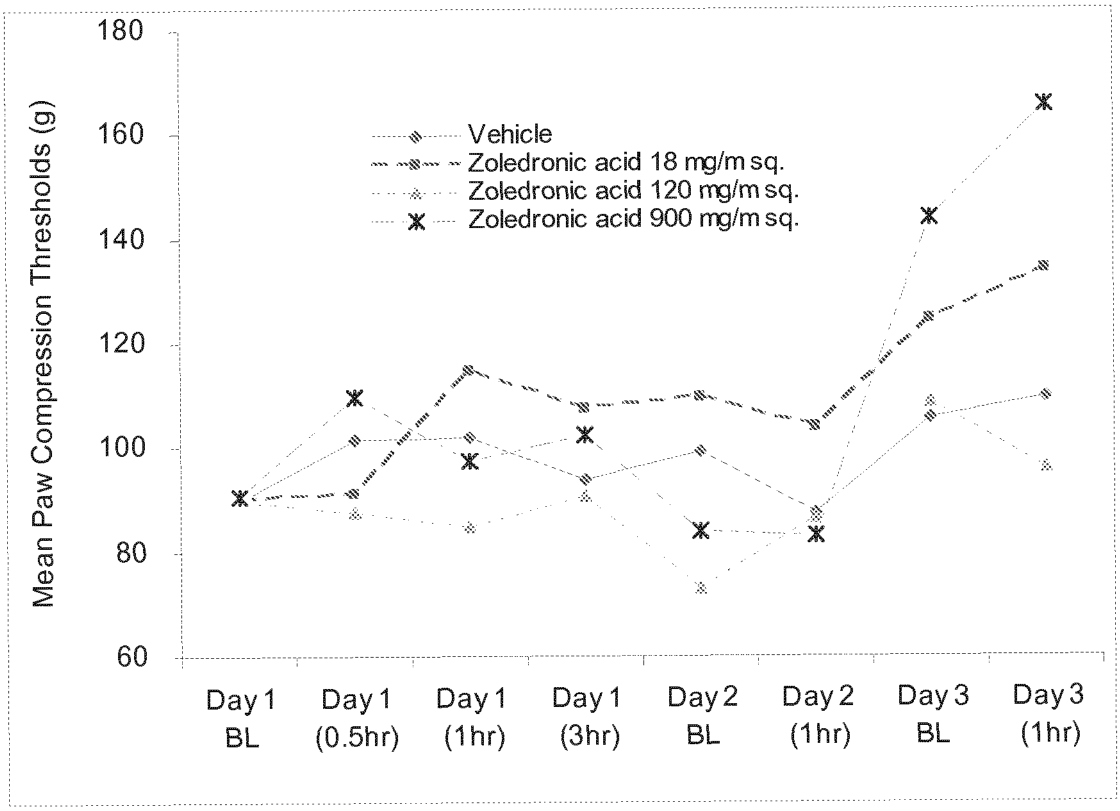
Mean Paw Compression Thresholds (g)
180
160
140
120
100
80
60
Vehicle
Zoledronic acid 18 mg/m sq.
Zoledronic acid 120 mg/m sq.
Zoledronic acid 900 mg/m sq.
Day 1 BL
Day 1 (0.5hr)
Day 1 (1hr)
Day 1 (3hr)
Day 2 BL
Day 2 (1hr)
Day 3 BL
Day 3 (1hr)

FIG. 1

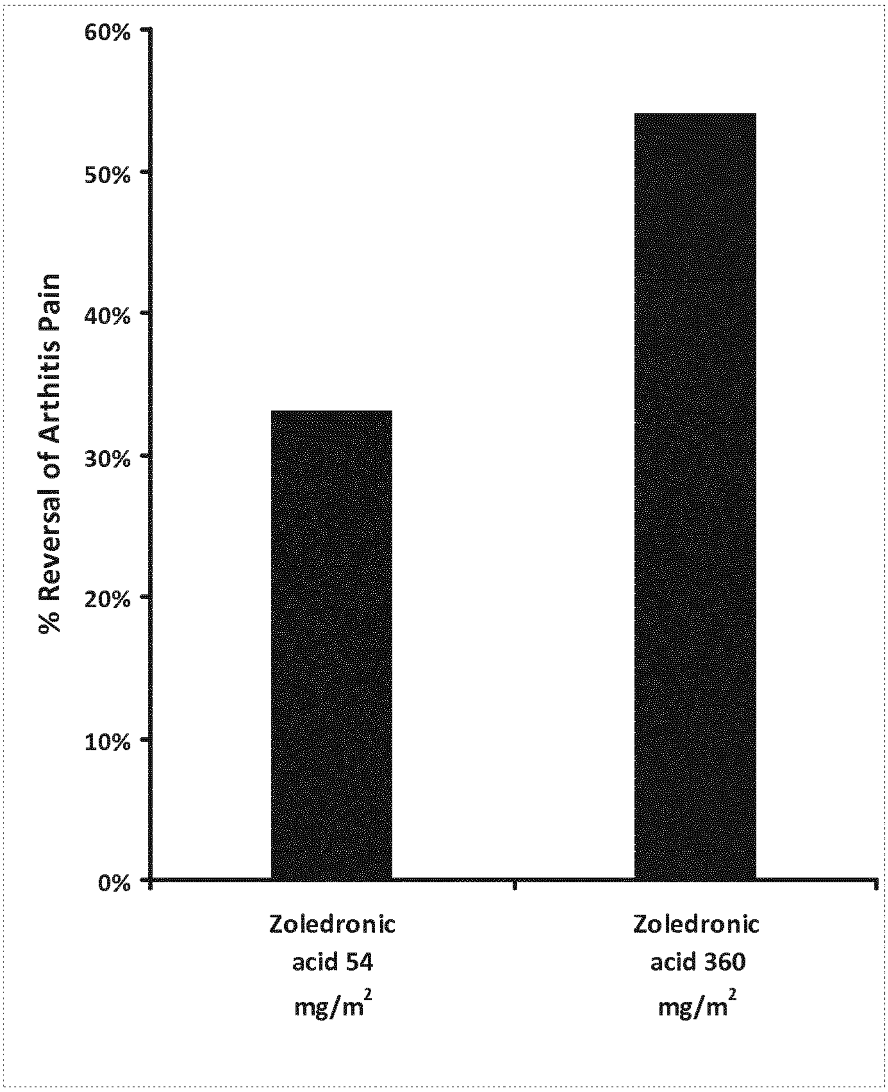
% Reversal of Arthitis Pain
60%
50%
40%
30%
20%
10%
0%
Zoledronic acid 54 mg/m2
Zoledronic acid 360 mg/m2

FIG. 2A

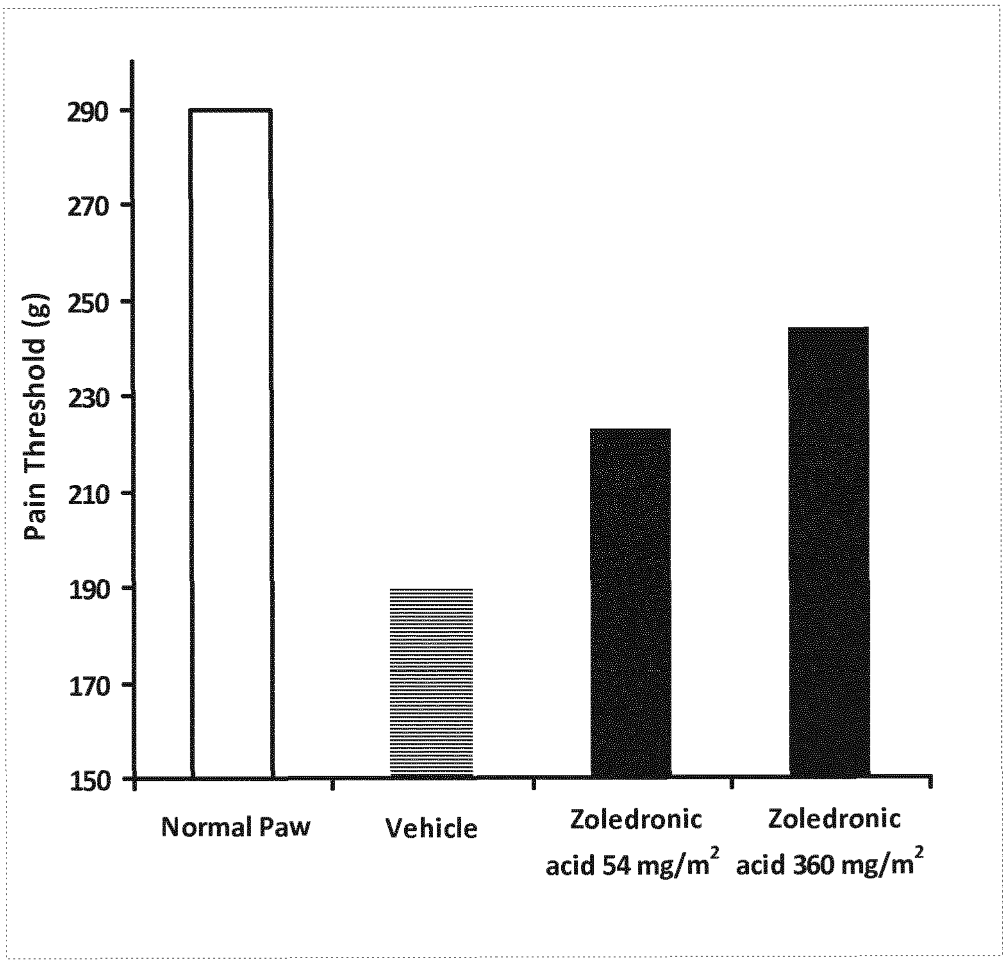
Pain Threshold (g)
290
270
250
230
210
190
170
150
Normal Paw
Vehicle
Zoledronic acid 54 mg/m2
Zoledronic acid 360 mg/m2

FIG. 2B

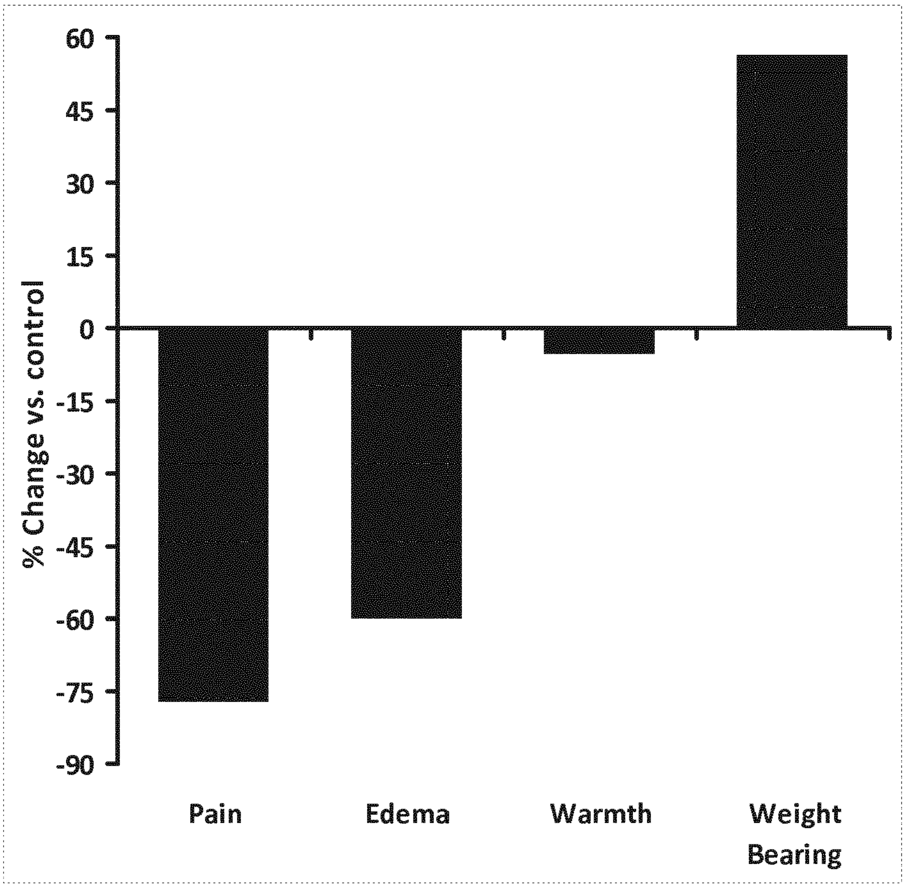
60
45
30
15
0
-15
-30
-45
-60
-75
-90
% Change vs. control
Pain
Edema
Warmth
Weight Bearing

FIG. 3

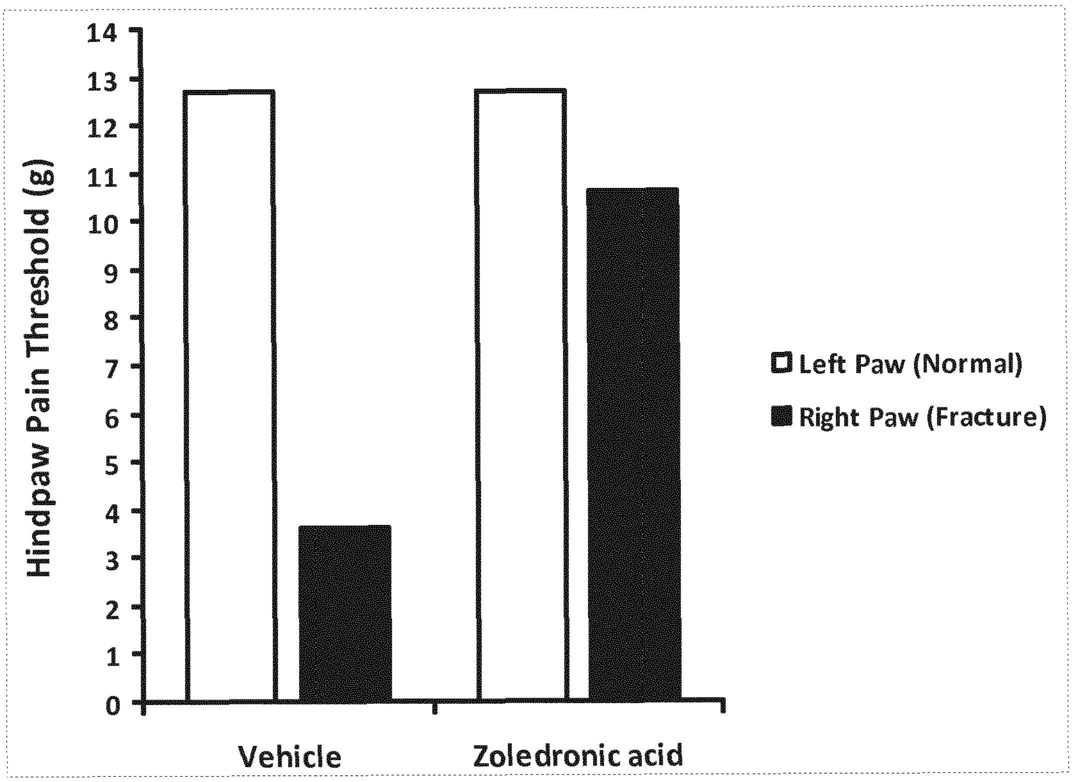
Hindpaw Pain Threshold (g)
14
13
12
11
10
9
8
7
6
5
4
3
2
1
0
Vehicle
Zoledronic acid
Left Paw (Normal)
Right Paw (Fracture)

FIG. 4

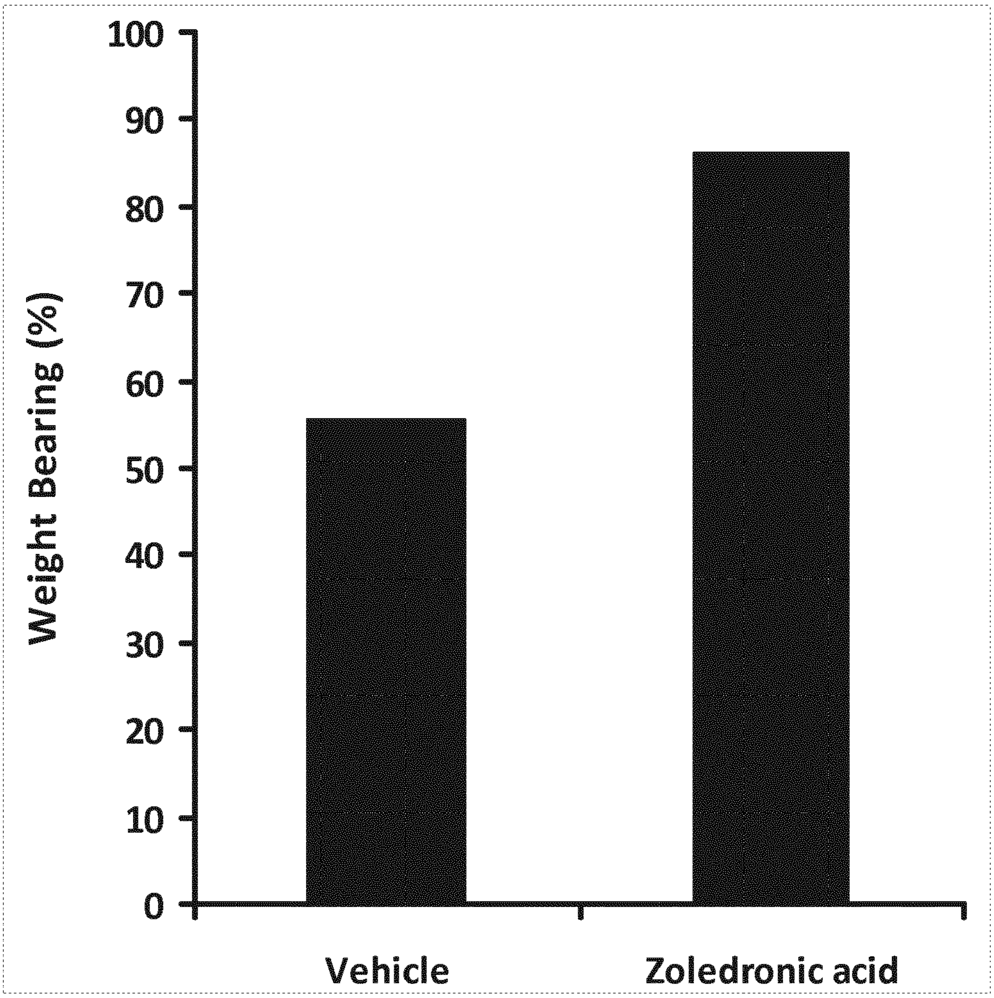
100
90
80
70
60
50
40
30
20
10
0
Weight Bearing (%)
Vehicle
Zoledronic acid

FIG. 5

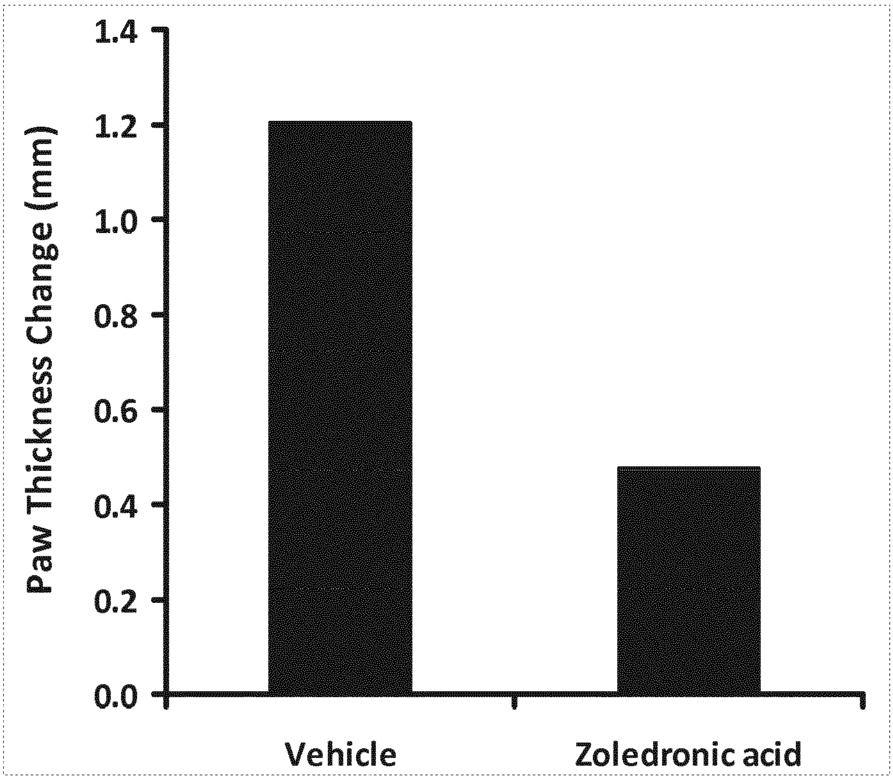
1.4
1.2
1.0
0.8
0.6
0.4
0.2
0.0
Paw Thickness Change (mm)
Vehicle
Zoledronic acid

FIG. 6

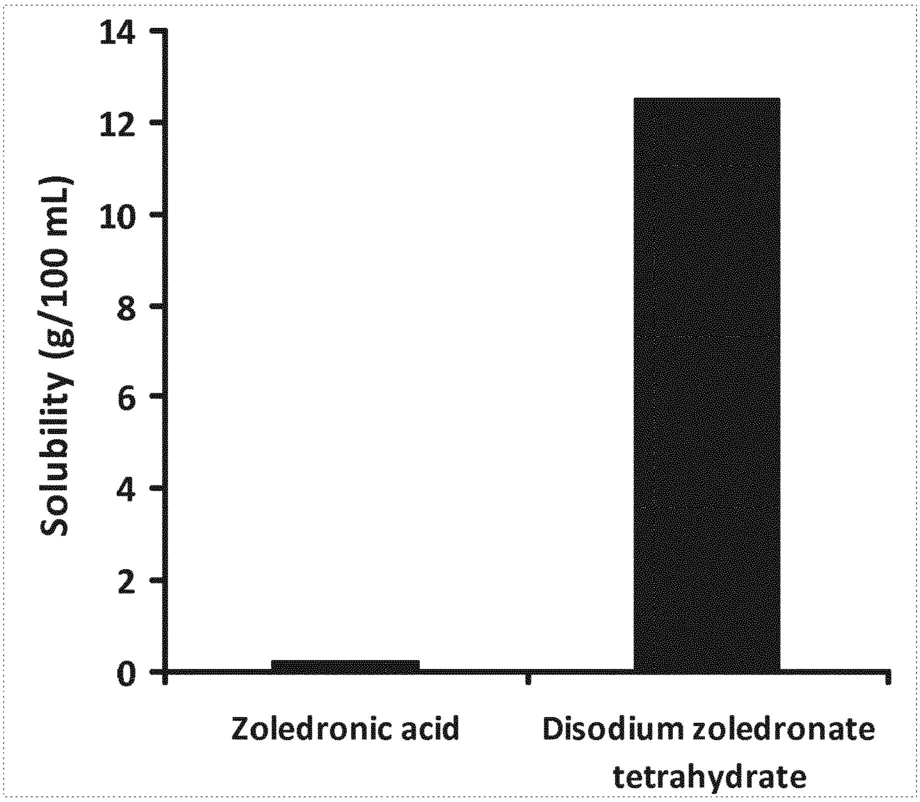
Solubility (g/100 mL)
14
12
10
8
6
4
2
0
Zoledronic acid
Disodium zoledronate tetrahydrate

FIG. 7

Europäisches Patentamt
European Patent Office
Office européen des brevets

# EUROPEAN SEARCH REPORT

Application Number
EP 17 20 6293

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X<br>Y | US 2010/215743 A1 (LEONARD THOMAS W [US]) 26 August 2010 (2010-08-26)<br>* examples 1-2 *<br>----- | 1,4-10<br>1-10 | INV.<br>A61K31/675<br>A61P29/00 |
| X<br>Y | WO 02/43738 A2 (NOVARTIS AG [CH]; NOVARTIS ERFIND VERWALT GMBH [AT]; FOX ALYSON [GB];) 6 June 2002 (2002-06-06)<br>* claims 1, 4, 5, 6, 9 *<br>* page 2, paragraph 2 *<br>* page 3, paragraphs 2, 3 *<br>* examples 5, 6 *<br>----- | 1,3-10<br>1-10 | |
| X<br>Y | US 2004/087551 A1 (PAPPAGALLO MARCO [US]) 6 May 2004 (2004-05-06)<br>* claims 1, 10 *<br>* paragraphs [0015], [0016], [0019], [0021] *<br>----- | 1,4-10<br>1-10 | |
| X<br>Y | CASTRO A.P.C. ET AL: "Zoledronic acid to treat complex regional pain syndrome type I in adult. Case report*",<br>REV DOR. SATILDE,<br>vol. 12, no. 1, January 2011 (2011-01),<br>XP055176375,<br>DOI: 10.1590/S1806-00132011000100015<br>* abstract *<br>* page 72, column 2, paragraph 2 *<br>-----<br>-/-- | 1,2,4-10<br>1-10 | TECHNICAL FIELDS SEARCHED (IPC)<br>A61K<br>A61P |

The present search report has been drawn up for all claims

1

| Place of search | Date of completion of the search | Examiner |
|---|---|---|
| Munich | 9 April 2018 | Peris Antoli, Berta |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 17 20 6293

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y,P | LASLETT LAURA LOUISE, ET AL: "Zoledronic acid reduces knee pain and bone marrow lesions over 1 year: a randomised controlled trial", ANNALS OF THE RHEUMATIC DISE, BRITISH MEDICAL ASSOCIATION, GB, vol. 71, no. 8, 31 August 2012 (2012-08-31), pages 1322-1328, XP008180591, ISSN: 0003-4967 * abstract * ----- | 1-10 | |
| Y | US 7 704 977 B2 (MERRION RES III LTD [IE]) 27 April 2010 (2010-04-27) * example 17 * ----- | 1-10 | |
| Y | MCHUGH CATHERINE ET AL: "MER-101 tablets: A pilot bioavailability study of a novel oral formulation of zoledronic acid", MOLECULAR CANCER THERAPEUTICS; AACR-NCI-EORTC INTERNATIONAL CONFERENCE: MOLECULAR TARGETS AND CANCER THERAPEUTICS-- OCT 22-26, 2007; SAN FRANCISCO, CA, AMERICAN ASSOCIATION FOR CANCER RESEARCH, US , vol. 6 November 2007 (2007-11), XP002750821, ISSN: 1535-7163 Retrieved from the Internet: URL:http://mct.aacrjournals.org/content/6/11_Supplement/B194.short [retrieved on 2015-11-11] * the whole document * ----- -/-- | 1-10 | TECHNICAL FIELDS SEARCHED (IPC) |

The present search report has been drawn up for all claims

1

| Place of search | Date of completion of the search | Examiner |
|---|---|---|
| Munich | 9 April 2018 | Peris Antoli, Berta |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 17 20 6293

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| T | "Equivalent Surface Area Dosage Conversion Factors", INTERNET CITATION, April 2015 (2015-04), page 1, XP002750822, Retrieved from the Internet: URL:https://ncifrederick.cancer.gov/lasp/acuc/frederick/Media/Documents/ACUC42.pdf [retrieved on 2015-11-10] ----- | | |

TECHNICAL FIELDS SEARCHED (IPC)

1

The present search report has been drawn up for all claims

| Place of search | Date of completion of the search | Examiner |
|---|---|---|
| Munich | 9 April 2018 | Peris Antoli, Berta |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

page 3 of 3

## ANNEX TO THE EUROPEAN SEARCH REPORT ON EUROPEAN PATENT APPLICATION NO. EP 17 20 6293

This annex lists the patent family members relating to the patent documents cited in the above-mentioned European search report. The members are as contained in the European Patent Office EDP file on The European Patent Office is in no way liable for these particulars which are merely given for the purpose of information.

09-04-2018

| Patent document cited in search report | Publication date | Patent family member(s) | Publication date |
|---|---|---|---|
| US 2010215743 A1 | 26-08-2010 | AR 075613 A1 | 20-04-2011 |
| | | CA 2751854 A1 | 02-09-2010 |
| | | EP 2400851 A1 | 04-01-2012 |
| | | JP 2012518686 A | 16-08-2012 |
| | | JP 2016104759 A | 09-06-2016 |
| | | TW 201035109 A | 01-10-2010 |
| | | US 2010215743 A1 | 26-08-2010 |
| | | WO 2010099255 A1 | 02-09-2010 |
| WO 0243738 A2 | 06-06-2002 | AT 366112 T | 15-07-2007 |
| | | AU 1706102 A | 11-06-2002 |
| | | AU 2002217061 B2 | 28-04-2005 |
| | | BR 0115696 A | 10-02-2004 |
| | | CA 2427161 A1 | 06-06-2002 |
| | | CN 1535152 A | 06-10-2004 |
| | | CZ 20031467 A3 | 17-09-2003 |
| | | DE 60129242 T2 | 13-03-2008 |
| | | DK 1339411 T3 | 29-10-2007 |
| | | EP 1339411 A2 | 03-09-2003 |
| | | ES 2287191 T3 | 16-12-2007 |
| | | HU 0302556 A2 | 28-11-2003 |
| | | IL 155363 A | 13-04-2008 |
| | | JP 2004514696 A | 20-05-2004 |
| | | KR 20030057544 A | 04-07-2003 |
| | | KR 20080043409 A | 16-05-2008 |
| | | MX PA03004815 A | 25-09-2003 |
| | | NO 20032405 A | 27-05-2003 |
| | | NZ 525871 A | 29-04-2005 |
| | | PL 361774 A1 | 04-10-2004 |
| | | PT 1339411 E | 12-10-2007 |
| | | RU 2325913 C2 | 10-06-2008 |
| | | SK 6542003 A3 | 02-12-2003 |
| | | TW I275393 B | 11-03-2007 |
| | | US 2004063670 A1 | 01-04-2004 |
| | | US 2008207565 A1 | 28-08-2008 |
| | | WO 0243738 A2 | 06-06-2002 |
| | | ZA 200303247 B | 10-05-2004 |
| US 2004087551 A1 | 06-05-2004 | US 2004087551 A1 | 06-05-2004 |
| | | US 2011098252 A1 | 28-04-2011 |
| US 7704977 B2 | 27-04-2010 | AU 2007235251 A1 | 18-10-2007 |
| | | BR PI0710503 A2 | 16-08-2011 |
| | | CA 2648594 A1 | 18-10-2007 |
| | | CN 101437522 A | 20-05-2009 |
| | | CN 105232482 A | 13-01-2016 |

EPO FORM P0459

For more details about this annex : see Official Journal of the European Patent Office, No. 12/82

## ANNEX TO THE EUROPEAN SEARCH REPORT ON EUROPEAN PATENT APPLICATION NO. EP 17 20 6293

This annex lists the patent family members relating to the patent documents cited in the above-mentioned European search report.
The members are as contained in the European Patent Office EDP file on
The European Patent Office is in no way liable for these particulars which are merely given for the purpose of information.

09-04-2018

| Patent document cited in search report | Publication date | Patent family member(s) | Publication date |
|---|---|---|---|
| | | EP 2007397 A2 | 31-12-2008 |
| | | EP 2526950 A1 | 28-11-2012 |
| | | ES 2426445 T3 | 23-10-2013 |
| | | IL 194277 A | 30-01-2014 |
| | | JP 5450052 B2 | 26-03-2014 |
| | | JP 2009533349 A | 17-09-2009 |
| | | JP 2014037412 A | 27-02-2014 |
| | | JP 2016040301 A | 24-03-2016 |
| | | KR 20090037851 A | 16-04-2009 |
| | | RU 2008144136 A | 20-05-2010 |
| | | US 2007238707 A1 | 11-10-2007 |
| | | US 2010022480 A1 | 28-01-2010 |
| | | US 2010247640 A1 | 30-09-2010 |
| | | WO 2007117706 A2 | 18-10-2007 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 61646538 B
- US 61647478 B
- US 61654292 B
- US 61654383 B
- US 61655527 B
- US 61655541 B
- US 61764563 B
- US 61762225 B
- US 61767647 B
- US 61767676 B
- US 61803721 B


### Non-patent literature cited in the description


- *Remington's Pharmaceutical Sciences,* 2005
- **GUO TZ et al.** *Pain.,* 2004, vol. 108, 95-107
- **KINGERY WS et al.** *Pain,* 2003, vol. 104, 75-84